# EUROPEAN PATENT SPECIFICATION

(11) **EP 4 622 707 B1**
(45) Date of publication and mention of the grant of the patent: **25.03.2026**
(21) Application number: 23707438.0
(22) Date of filing: 24.01.2023
(51) Int. Cl.: A61N 2/02, A61N 1/37, G01R 29/08

(54) **PULSED ELECTROMAGNETIC FIELD DEVICE WITH SENSOR**
GEPULSTES ELEKTROMAGNETISCHES FELDGERÄT MIT SENSOR
DISPOSITIF DE CHAMP ÉLECTROMAGNÉTIQUE PULSÉ AVEC CAPTEUR

(43) Date of publication of application: 01.10.2025
(73) Proprietor: Hofmeir Magnetics Limited, Reading RG1 1DA (GB)
(72) Inventor: BEDDARD, Paul, Hertfordshire HR13NP (GB)
(74) Representative: Galdagon, Atheer Fakhreldin Ali
(86) International application number: PCT/GB2023/050148
(87) International publication number: WO 2024/156973

(56) References cited:
- EP-B1- 2 590 711
- WO-A1-2015/070259
- GB-A- 2 602 669
- US-A1- 2021 370 086

## Description

### TECHNICAL FIELD

The present disclosure relates to systems and methods for generating pulsed electromagnetic fields, particularly, but not exclusively, for generating energy pulses for providing physiological effects on a human or animal body.

### BACKGROUND

Pulsed electromagnetic fields can be used to provide physiological effects on the body. For example, pulsed electromagnetic fields can be used to provide therapeutic benefits, such as treating ailments like joint and muscle pain, and assisting with the healing of broken bones and fractures.

Pulsed electromagnetic field devices (PEMF devices) are used to generate pulsed electromagnetic fields. A pulsed electromagnetic field device may generate a current or voltage signal having the desired pulsed waveform. The signal is outputted to a coil, e.g. a coil looped inductor. The coil then generates a corresponding pulsed electromagnetic field. The pulsed electromagnetic field may have a very high energy. For example, the device may generate the pulsed electromagnetic field using high electrical powers, for example on the scale of kilo-volts/amps. Therefore, there is a risk that the pulsed electromagnetic field may cause damage to electronic and electrical circuitry that is in proximity to the coil, including the device's own circuitry.

It is desirable to develop a pulsed electromagnetic field device that overcomes or mitigates the problems associated with generating high energy pulsed electromagnetic fields.

The following document is relevant: EP 2 590 711 B1 which discusses a portable, noninvasive device for providing therapeutic treatment to a joint to promote healing of the joint including a cuff positionable around the joint. The cuff includes first and second therapeutic units, each including a signal generator, an electromagnetic stimulator configured to provide an electromagnetic field within the joint, and a single-use thermal exchange component configured to provide temperature-based therapy to the joint.

### SUMMARY OF THE DISCLOSURE

The invention is outlined by the appended independent claims.

The present disclosure relates to a pulsed EMF (PEMF) device with a safety feature that prevents the device from damaging electronic circuitry that is in proximity to the coil looped inductor. More particularly, the safety feature may prevent the EMF pulses outputted by the coil looped inductor from damaging the device's own internal circuitry. In normal operation, the device generates sequences of EMF pulses when the device is powered on, and outputs the EMF pulses at the coil looped inductor. The device also includes a pulsed EMF sensor. The pulsed EMF sensor detects EMF pulses within the vicinity of the device. If the pulsed EMF sensor detects one or more EMF pulses, the device will stop generating EMF pulses, even though the device is powered on. As such, the coil looped inductor will stop outputting EMF pulses. Therefore, the device may preserve its own internal circuitry in situations when the coil looped inductor and/or the EMF pulses outputted by the coil looped inductor are in a close proximity to the device's internal circuitry.

In a first aspect of the present disclosure, there is provided a pulsed electromagnetic field (EMF) device comprising:
a pulsed EMF generator configured to generate EMF pulses; and
a sensor having an output in communication with the pulsed EMF generator, wherein the sensor is configured to detect an EMF pulse and provide, over the output, an output signal indicative of each detected EMF pulse,
wherein the pulsed EMF generator is further configured to stop generating the EMF pulses in response to the sensor detecting one or more EMF pulses.

Advantageously, the pulsed EMF device can stop or be prevented from generating the EMF pulses in order to preserve and protect its own internal circuitry. For example, the pulsed EMF generator may be configured to prevent the generating of EMF pulses in response to the sensor detecting one or more EMF pulses. Due to human error, the EMF pulses generated by the pulsed EMF generator may feed back to the pulsed EMF device. For example, the pulsed EMF generator may comprise a handheld inductor coil that outputs the EMF pulses. If the inductor coil is positioned too close to the rest of the pulsed EMF device, then the EMF pulses can damage other circuitry in the pulsed EMF device, such as the rest of the circuitry of the pulsed EMF generator. As such, by including the sensor in the pulsed EMF device, the pulsed EMF generator can stop the generating of the EMF pulses in response to the sensor detecting one or more EMF pulses, thereby protecting the internal circuitry of the pulsed EMF device.

In exemplary embodiments each EMF pulse comprises a decaying sequence of electromagnetic (EM) oscillations. Advantageously, this type of EMF pulse has been found to more effectively provide a physiological effect on the body.

In exemplary embodiments, the output of the sensor is in communication with the pulsed EMF generator over a fibre optic cable, and the output signal of the sensor is a light signal.

Advantageously, using a fibre optic cable and a light signal to deliver the output of the sensor to the pulsed EMF generator can reduce the electrostatic sensitivity of the pulsed EMF device. In particular, it has been found that using an electrical signal to deliver the output of the sensor may cause large amounts of static electricity in the device. Moreover, a larger amount of static electricity is produced for larger lengths of electrical cable. Therefore, using a fibre optic cable can reduce the effect of static electricity and allow for further distances between the sensor and the circuitry of the pulsed EMF generator. Advantageously, the circuitry of the pulsed EMF generator can be kept at further distance from the sensor for better isolation and protection. Moreover, use of a fibre optic cable may allow the PEMF device to be compliant with industry standards and pass electromagnetic compatibility (EMC) tests.

In exemplary embodiments, the sensor comprises a coil loop configured to induce a current in response to the EMF pulse.

Advantageously, the sensor is able to detect and convert a detected EMF pulse into an electrical current signal, and convert the electrical signal into a light signal for transporting over the fibre optic cable.

In exemplary embodiments, the sensor comprises a conditioning circuit coupled to the coil loop, wherein the conditioning circuit is configured to condition a waveform of the induced current and output a conditioned current.

Advantageously, by appropriately conditioning the electrical current signal, the electrical current signal can be made to comprise a smoothed pulse for each pulse of the detected EMF (i.e. for each sequence of damped oscillations of the detected EMF). Consequently, the signal outputted by the sensor (e.g. a light signal) will comprise a smoothed pulse for each pulse of the detected EMF. Advantageously, the pulsed EMF generator can easily determine whether the sensor has detected an EMF pulse, and how many EMF pulses have been detected.

In exemplary embodiments, the sensor comprises an optical transmitter configured to convert the conditioned current into the light signal and supply the light signal to the fibre optic cable.

Advantageously, the output signal of the sensor is provided in a suitable form for use with the fibre optic cable. In some embodiments, the conditioning circuit is optional, and the optical transmitter can convert the induced current into the light signal.

In exemplary embodiments, the conditioning circuit comprises a rectifier configured to at least partially rectify the induced current and output a rectified current.

In exemplary embodiments, the conditioning circuit comprises a low pass filter configured to filter the rectified current or the induced current.

Advantageously, the rectification and low pass filtering causes the current signal to comprise a smoothed pulse for each EMF pulse (i.e. for each sequence of decaying oscillations in the detected EMF).

In exemplary embodiments, the conditioning circuit comprises a suppression circuit that is configured to limit a current and/or a voltage in the conditioning circuit.

The sensor initially receives a high electrical power due to the high power level of the detected EMF. Advantageously, the suppression circuit can limit the current and/or voltage levels in the conditioning circuit in order to improve the reliability of the sensor and prevent damage to the components of the sensor.

In exemplary embodiments the rectifier comprises at least one diode.

Advantageously, the rectifier can be a half-wave rectifier or a full wave rectifier. More advantageously, if the rectifier is a half-wave rectifier, the rectifier can be implemented using just one diode. This allows the sensor to maintain a small size and footprint, which is important for better integrating the sensor into the pulsed EMF device.

In exemplary embodiments the low pass filter comprises a first resistor and at least one capacitor arranged in a low pass filter configuration, optionally wherein the first resistor is arranged in series with the diode.

In some embodiments, the low pass filter comprises at least two capacitors, each being arranged in parallel with one another. By using two parallel capacitors in the low pass filter, the required capacitance of the low pass filter can be achieved using two smaller capacitors instead of one large capacitor. This is advantageous because the two smaller and parallel connected capacitors will have a smaller height in comparison to a single capacitor of an equivalent capacitance, which helps to minimise the total size of the sensor for improved integration of the sensor within the pulsed EMF device. Moreover, by using two capacitors, the sensor is made more reliable, since if one of the capacitors fail then the low pass filter may still utilise the remaining capacitor.

In exemplary embodiments, the suppression circuit comprises a transient voltage suppression (TVS) diode optionally arranged in parallel with the capacitor, a first LED arranged in parallel with the TVS diode, and a second resistor arranged in series with an output of the suppression circuit.

The TVS diode can advantageously clip or limit the voltage produced at the output of the low pass filter, thereby reducing the voltage to levels that are more tolerable to the components in the sensor. Furthermore, the first LED can also contribute to limiting the voltage. Additionally, the second resistor can limit the current outputted by the suppression circuitry to the optical transmitter, and thereby control the sensitivity of the optical transmitter.

In exemplary embodiments, the sensor further comprises a quenching circuit configured to provide a current path for non-rectified portions of the induced current. Optionally, the quenching circuit comprises a second diode and a resistance arranged in series between terminals of the coil loop.

The second diode preferably has a cathode coupled to the same terminal of the coil loop as an anode of the diode of the rectifier. Advantageously, the quenching circuit (e.g. the second diode and the resistance) operates to conduct and quench negative going pulses of the electrical waveform induced at the coil loop by the detected EMF. Consequently, the diode of the rectifier can be cheaper, smaller and/or have a lower tolerance value, since it does not have to withstand high voltages that occur during the negative parts of the waveform. This furthers the ability to provide a pulsed EMF sensor that can be easily integrated into a PEMF device.

In exemplary embodiments, the first LED is a visible light (e.g. white light) LED. Advantageously, the first LED can be used as a visible indicator to a user as to whether the sensor is detecting EMF pulses.

In exemplary embodiments, the optical transmitter comprises an infrared LED. Advantageously, the optical transmitter can reliably transmit the light signal to the pulsed EMF generator over the fibre optic cable.

In exemplary embodiments, the fibre optic cable is a polymer optical fibre. Advantageously, the fibre optic cable is more robust to bending and stretching in comparison to other types of fibre optic cable, such as glass fibre optic.

In exemplary embodiments, the pulsed EMF generator comprises a current generating circuit configured to generate a pulsed current.

In exemplary embodiments, the pulsed EMF generator comprises a coil looped inductor configured to output the EMF pulses in response to the pulsed current.

In exemplary embodiments, the pulsed EMF generator comprises control circuitry configured to control the current generating circuit to generate the pulsed current,
wherein the control circuitry is configured to receive the output signal from the sensor and stop the current generating circuit from generating the pulsed current in response to the sensor detecting one or more EMF pulses.

The control circuitry may be electrically sensitive. The electrically sensitive circuitry may be circuitry that controls the PEMF circuitry, such as that controls the current generating circuit. For example the control circuitry may comprise one or more microcontrollers and/or microprocessors to control the opening and closing of one or more switches in the current generating circuit. Therefore, when the control circuitry receives an indication that the sensor has detected one or more EMF pulses, the control circuitry can preserve its own circuitry by stopping the pulsed EMF device from generating further EMF pulses. Preferably, the control circuitry stops the current generating circuit from generating the pulsed current after receiving an indication that the sensor has sensed two or more consecutive EMF pulses (i.e. at least two light pulses are received).

In exemplary embodiments, the pulsed EMF device further comprises a housing, the housing containing at least the current generating circuit and the control circuitry of the pulsed EMF generator, wherein the sensor is embedded in a surface of the housing.

Advantageously, the sensor is positioned closer to the source of the potentially damaging EMF pulses. Preferably, the sensor is embedded in a top surface of the housing where a user is most likely to place or rest the handheld inductor coil.

In a second aspect of the present disclosure, there is provided a method comprising:
generating, using a pulsed EMF generator, a plurality of EMF pulses;
detecting, using a sensor, one or more EMF pulses;
communicating, from the sensor to the pulsed EMF generator, an output signal indicative of each detected EMF pulse; and
in response to the sensor detecting the one or more EMF pulses, stopping the pulsed EMF generator from generating further EMF pulses.

In exemplary embodiments, each EMF pulse comprises a decaying sequence of electromagnetic (EM) oscillations.

In exemplary embodiments, the output signal is a light signal and the light signal is communicated from the sensor to the pulsed EMF generator via a fibre optic cable.

In exemplary embodiments, detecting the one or more EMF pulses comprises inducing a current in a coil loop in response to the EMF pulse.

In exemplary embodiments, detecting the one or more EMF pulses comprises conditioning a waveform of the induced current and outputting a conditioned current using a conditioning circuit.

In exemplary embodiments, detecting the one or more EMF pulses comprises converting the conditioned current into the light signal using an optical transmitter, and supplying the light signal to the fibre optic cable.

In exemplary embodiments, conditioning the waveform of the induced current comprises at least partially rectifying the induced current using a rectifier to produce a rectified current.

In exemplary embodiments, conditioning the waveform of the induced current comprises low pass filtering the rectified current or the induced current.

In exemplary embodiments, conditioning the waveform of the induced current comprises limiting a current and/or a voltage at the output of the conditioning circuit.

In exemplary embodiments, the method further comprises quenching negative parts of the waveform of the induced current.

In exemplary embodiments, generating the EMF pulses using the pulsed EMF generator comprises:
controlling a current generating circuit using control circuitry to generate a pulsed current from the current generating circuit; and
outputting the EMF pulses from a coil looped inductor in response to the pulsed current,
wherein the control circuitry receives the output signal from the sensor and stops the current generating circuit from generating the pulsed current in response to the sensor detecting one or more EMF pulses.

In a third aspect of the present disclosure, there is provided a pulsed electromagnetic field (EMF) sensor comprising:
a coil loop configured to induce a current in response to a EMF pulse,
a conditioning circuit coupled to the coil loop, wherein the conditioning circuit is configured to condition a waveform of the induced current and output a conditioned current, and
an optical transmitter configured to convert the conditioned current into a light signal.

In exemplary embodiments, the pulsed EMF sensor further comprises a fibre optic cable, wherein the optical transmitter is configured to supply the light signal to the fibre optic cable.

In exemplary embodiments, the conditioning circuit comprises a rectifier configured to at least partially rectify the induced current and output a rectified current.

In exemplary embodiments, the conditioning circuit comprises a low pass filter configured to filter the rectified current or the induced current.

In exemplary embodiments, the conditioning circuit comprises a suppression circuit that is configured to limit a current and/or a voltage at the output of the conditioning circuit.

In exemplary embodiments, the rectifier comprises at least one diode.

In exemplary embodiments, the low pass filter comprises a first resistor and at least one capacitor arranged in a low pass filter configuration, optionally wherein the first resistor is arranged in series with the diode.

In exemplary embodiments, the suppression circuit comprises a transient voltage suppression (TVS) diode optionally arranged in parallel with the capacitor, a first LED arranged in parallel with the TVS diode, and a resistor arranged in series with an output of the suppression circuit.

In exemplary embodiments, the pulsed EMF sensor comprises a second diode and a resistance arranged in series between terminals of the coil loop.

In exemplary embodiments, the first LED is a visible light LED.

In exemplary embodiments, the optical transmitter comprises an infrared LED.

In exemplary embodiments, the fibre optic cable is made of a polymer optical fibre.

In exemplary embodiments, the sensor further comprises a printed circuit board (PCB), and wherein the coil loop, the conditioning circuit and at least part of the suppression circuit are provided on the same major surface of the PCB. Advantageously, by using just one of the major surfaces of the PCB, the overall size and footprint of the sensor can be minimise to provide for better integration of the sensor into a pulsed EMF device.

In exemplary embodiments, the pulsed EMF sensor further comprises a protective cover over the major surface.

### BRIEF DESCRIPTION OF THE DRAWINGS

Examples of the present disclosure are now described with reference to the accompanying drawings, in which:
Figure 1 shows a pulsed electromagnetic field therapy device including a pulsed electromagnetic field sensor according to an example of the present disclosure;
Figure 2 shows an oscilloscope trace showing a current through an inductor of the pulsed electromagnetic field therapy device of Figure 1, as a function of time;
Figure 3 shows a pulsed electromagnetic field sensor according to an example of the present disclosure;
Figure 4 shows a circuit level schematic of a pulsed electromagnetic field sensor according to an example of the present disclosure;
Figure 5 shows an oscilloscope trace showing current at a location in the pulsed electromagnetic field sensor, as a function of time;
Figure 6 shows an oscilloscope trace showing current at another location in the pulsed electromagnetic field sensor, as a function of time;
Figure 7 shows a perspective view of an implementation of the pulsed electromagnetic field sensor according to an example of the present disclosure;
Figure 8 shows a plan view of an implementation of the pulsed electromagnetic field sensor according to an example of the present disclosure;
Figure 9 shows a first example of a pulsed electromagnetic field circuit for use in a pulsed electromagnetic field device;
Figure 10 shows a second example of a pulsed electromagnetic field circuit for use in a pulsed electromagnetic field device;
Figure 11 shows an oscilloscope trace showing current in a resonant circuit of the current generation circuit of Figure 10, as a function of time;
Figure 12 shows a third example of a pulsed electromagnetic field circuit for use in a pulsed electromagnetic field device; and
Figure 13 shows a circuit level schematic of a pulsed electromagnetic field sensor according to another example of the present disclosure.

### DETAILED DESCRIPTION

### Pulsed Electromagnetic Field Device with PEMF Sensor

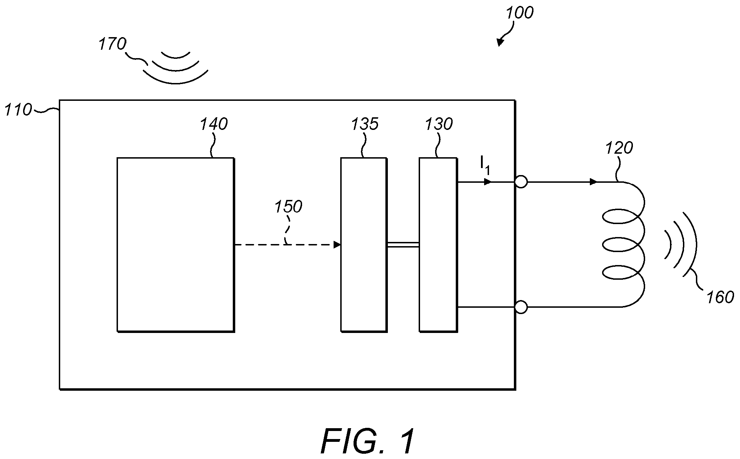

Figure 1 shows a pulsed electromagnetic field (PEMF) device 100 according to an example of the present disclosure. The PEMF device 100 is configured to generate and emit a pulsed electromagnetic field (PEMF) 160. The PEMF device 100 is also configured to detect a PEMF (e.g. the PEMF 170), and stop the generating and/or emitting of the PEMF 120 if a PEMF is detected.

The PEMF device 100 comprises a housing 110 and a coil looped inductor 120. The housing contains a current generating circuit 130, control circuitry 135 and a PEMF sensor 140. The coil looped inductor 120 is electrically coupled to an output of the current generating circuit 130. The control circuitry 135 is electrically coupled to the current generating circuit 130. An output of the PEMF sensor 140 is coupled to the control circuitry 135 via a fibre-optic cable 150.

The coil looped inductor 120 is coupled externally to the housing. The coil looped inductor 120 is movable through space relative to the housing 110. For example, if the PEMF device 100 is used for PEMF therapy, a user or operator can freely move the coil looped inductor 120 to an area of a body to be treated. As such, the coil looped inductor 120 can be handheld during use of the PEMF device 100. Moreover, the coil looped inductor 120 can be placed adjacent to or around a part of a human or animal body (e.g. a limb or joint), in order to provide the pulsed EMF to that part of the body and produce a physiological effect. As such, the coil looped inductor 120 may be made of a suitably flexible material(s) so that it can be operated as described herein. The coil looped inductor 120 may also be suitably insulated so that it can be handheld and/or hand operated during use, e.g. whilst the coil looped inductor 120 is outputting the PEMF 160.

The current generating circuit 130 is configured to generate and supply a current I₁ to the coil looped inductor 120. The coil looped inductor 120 then generates and emits the pulsed EMF 160 in response to the current I₁ flowing through the coil looped inductor 120.

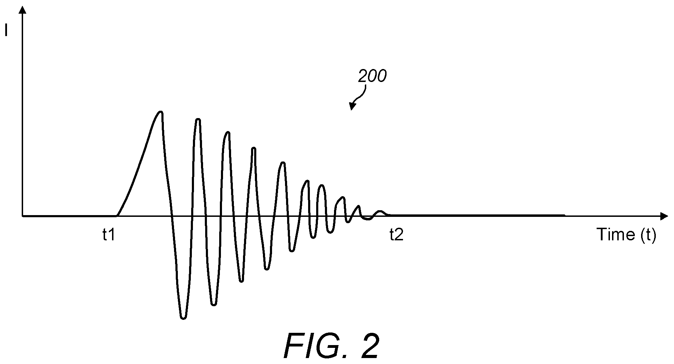

Figure 2 illustrates an oscilloscope trace 200 showing the current I₁ as a function of time t. As shown, the current I₁ is an alternating current (AC) having a decaying sinusoidal shape. In other words, the current I₁ comprises a sequence of decaying or damped sinusoidal oscillations. The sequence of oscillations start at time t₁ and end at time t₂. The current I₁ may have a peak value of 100 Amps, 1500 Amps, or any value within the range 100-1500 Amps. Furthermore, the length of time between times t₁ and t₂ may be 1 millisecond, 2 milliseconds, or any length of time between 1 and 2 milliseconds. However, any other peak currents and pulse times are within the scope of the present disclosure.

The coil looped inductor 120 will generate an EMF that is proportional to the current I₁. In particular, the EMF generated by the coil looped inductor 120 will be an alternating EMF comprising a sequence of decaying or damped sinusoidal electromagnetic field oscillations. As such, the energy waveform of the generated EMF may correspond to the trace 200 shown in Figure 2. The sequence of decaying sinusoidal electromagnetic oscillations, e.g. as shown in Figure 2, may correspond to one pulse of the pulsed EMF 160. As such, the pulsed EMF 160 may comprise a series of electromagnetic pulses, each pulse comprising a sequence of damped sinusoidal oscillations. The current generating circuit 130 may be configured to provide the current I₁ such that it repeats the shape 200 shown in Figure 2 in a series, so that the coil looped inductor 120 generates and emits a series of electromagnetic pulses comprising a sequence of damped sinusoidal oscillations. Preferably, the coil looped inductor 120 outputs approximately six pulses every second, wherein each pulse corresponds to the sequence of damped sinusoidal oscillations.

Reference is made back to Figure 1. The PEMF sensor 140 is configured to detect a PEMF (e.g. the PEMF 170). The PEMF sensor 140 is suitably sensitive to detect PEMFs within the proximity of the sensor 140. In some examples, the PEMF sensor 140 is capable of detecting PEMFs that have a strength of at least 200mT (millitesla) at an approximate range of 100mm. The PEMF sensor 140 is configured to generate an output signal that is indicative of whether a PEMF has been detected. Preferably, the output signal is in the form of infrared light. However, the output signal can be any other type of light, such as any visible light or ultra-violet light. In some examples, the PEMF sensor 140 will generate the output signal so that the output signal contains a pulse for each pulse of the detected PEMF. As discussed above, a pulse of the detected PEMF corresponds to a sequence of decaying or damped sinusoidal oscillations, as illustrated in Figure 2. Since the fibre optic cable 150 is coupled to the output of the PEMF sensor 140, the PEMF sensor 140 will provide its output signal to the fibre optic cable 150.

The control circuitry 135 is configured to control the operation of the current generation circuit 130. For example, the current generation circuit 130 may comprise one or more switches that require periodic opening and closing in order to generate the current I₁. The control circuitry 135 may be configured to control the opening and closing of said switches so that the current generation circuit 130 generates the current I₁ and consequently the coil looped inductor 120 generates the PEMF 160. Example implementations of the current generation circuit 130 are described in more detail below. Furthermore, the control circuitry 135, the current generating circuit 130 and the coil looped inductor 120 may be considered together as a PEMF generator.

The control circuitry 135 also receives the output signal of the PEMF sensor 140 over the fibre optic cable. The control circuitry 135 is further configured to stop the current generating circuit 130 from generating the current I₁ if the output signal indicates that the PEMF sensor 140 has detected a PEMF. In particular, the control circuitry 135 detects whether the output signal from the PEMF sensor 140 contains pulses. If one or more pulses are detected in the output signal from the PEMF sensor 140, the control circuitry 135 stops the current generating circuit 130 from generating the current I₁. In some examples, the control circuitry 135 stops the current generating circuit 130 upon detecting one pulse in the output signal from the PEMF sensor 140. Alternatively, the control circuitry 135 may wait to detect at least 2, 3, 4 or more pulses in the output signal before stopping the current generating circuit 130. In some examples, the control circuitry 135 can stop the current generating circuit 130 from generating the current I₁ by causing an open circuit in the current generating circuit 130, e.g. by opening a switch that is being used to generate the current I₁.

Preferably, the control circuitry 135 comprises one or more microcontrollers or microprocessors that are configured and arranged to perform the functions of the control circuitry 135 as described herein. In some examples, the control circuitry 135 may comprise an optical receiver that receives the output signal of the PEMF sensor 140 over the fibre optic cable 150, and then converts the output signal from a light signal to an electrical signal. The receiver may then provide the electrical signal to the microcontroller(s) or microprocessor(s) to perform the functions described herein.

Although not shown in Figure 1, the PEMF device 100 also comprises a power supply and/or means for coupling the PEMF device 100 to a power supply. For example, the PEMF device 100 may comprise a battery that supplies power to the rest of the device, such as to the control circuitry 130 and the current generating circuitry 130. Alternatively or additionally, the PEMF device 100 comprises a plug or cable for connecting to an external power supply, such as to an external battery or a mains power supply.

The electronic circuitry in the PEMF device 100 is highly sensitive to the PEMF 160 that is generated by the coil looped inductor 120. The control circuitry 135 (e.g. the microcontrollers or microprocessors) is particularly sensitive to PEMFs. Therefore, if the coil looped inductor 120 is in close proximity to the control circuitry 135, then the PEMF 160 being generated by the coil looped inductor 120 can cause irreparable and permanent damage to the control circuitry 135. This can occur during use, for example if a user or operator rests the coil looped inductor 120 on the housing 110, close to the control circuitry 135, whilst the PEMF device 100 is powered on.

Therefore, the PEMF device 100 advantageously includes the PEMF sensor 140. If a PEMF is detected, the PEMF sensor 140 causes the control circuitry 135 to stop the current generating circuit 130 from generating of the current I₁, which thereby stops the generation of the PEMF 160. Consequently, the PEMF 160 is turned off before it can cause damage to the control circuitry 135.

Preferably, the PEMF sensor 140 is positioned close to a surface of the housing 110 (e.g. close to a top surface of the housing 110) and the control circuitry 135 is positioned at a distance to said surface of the housing 110. For example, if the PEMF sensor 140 is positioned under or near to the top surface of the housing 110, then the control circuitry 135 may be positioned as far as possible from the PEMF sensor 140. Consequently, as a user begins to place the coil looped inductor 120 on the top surface of the housing 110, then the PEMF sensor 140 will detect the PEMF 160 and cause the PEMF 160 to turn off before the coil looped inductor 120 is close enough to the control circuitry 135 to cause permanent damage. More preferably, the control circuitry 135 is maximally distanced from the PEMF sensor 140 and the top surface of the housing 110, within the design limits of the PEMF device 100, to minimise risk of damage to the control circuitry 135. In another example, the PEMF sensor 140 may be embedded in the top surface of the housing 110.

It should be appreciated that this is one possible physical arrangement of the PEMF sensor 140 and the control circuitry 135 and any other physical arrangement is within the scope of the present disclosure. In other examples, the PEMF sensor 140 and the control circuitry 135 can be arranged such that the PEMF sensor 140 is positioned in between a surface of the housing 110 (e.g. a top surface) and the control circuitry 135, and this can be sufficient to reduce the risk of damage to the control circuitry 135 as the coil looped inductor 120 is brought in proximity to said surface.

As described above, the output of the PEMF sensor 140 is provided as a light signal to the control circuitry 135 via an fibre optic cable 150. Advantageously, the fibre-optic cable isolates the sensor 140 from damaging differential static voltage build ups. This may prevent the PEMF device 100 from generating large amounts of damaging static electricity. In particular, it has been observed that if the output of the PEMF sensor 140 is provided to the control circuitry 135 as an electrical (e.g. voltage or current) signal using electrical wire, then this generates large amounts of static electricity. The static electricity can build up to the region of several kilo-volts. The problem of static electricity build up increases as the length of the cable between the PEMF sensor 140 and the control circuitry 135 is increased. As such, the use of a fibre optic cable 150 and light signals allows the PEMF device 100 to have reduced electrostatic sensitivity. Moreover, a long length of the fibre optic cable 150 can be used to maximise the distance between the PEMF sensor 140 and the control circuitry 135 without generating large amounts of static electricity. Ultimately, this may allow the PEMF device to be compliant with industry standards pass electromagnetic compatibility (EMC) tests.

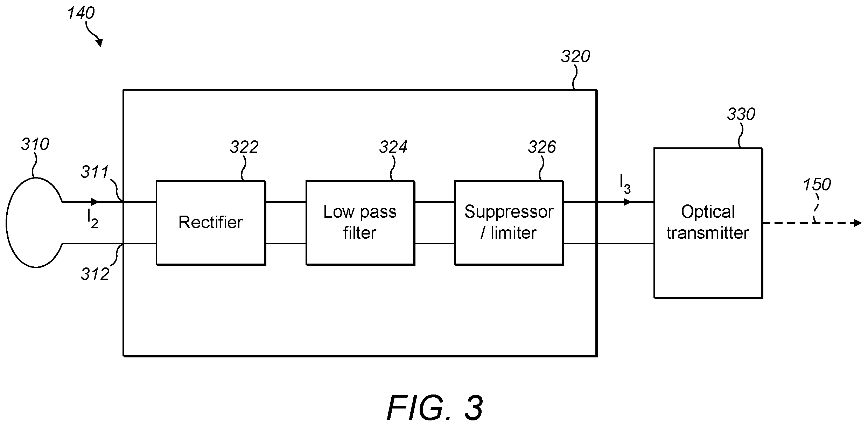

Figure 3 illustrates the components of the PEMF sensor 140 in more detail. The PEMF sensor 140 comprises a coil loop 310, a conditioning circuit 320 and an optical transmitter 330. The coil loop 310 comprises a first terminal 311 and a second terminal 312. The coil loop 310 is electrically coupled to an input of the conditioning circuit 320. In particular, the first terminal 311 and the second terminal 312 are coupled to the input of the conditioning circuit 320. The optical transmitter 330 is coupled to an output of the conditioning circuit 320.

In the presence of the PEMF 160, the coil loop 310 will induce a voltage or a potential difference across its terminals 311 and 312 in response to the pulsed EMF 160. The induced voltage will be an AC or alternating voltage corresponding to the pulsed EMF 160 and the current I₁ in the coil looped inductor 120. In particular, the induced voltage may comprise a sequence of damped or decaying sinusoidal oscillations, in correspondence with the shape of the pulsed EMF 160 and the current I₁. Consequently, a current I₂ is induced through the coil loop 310 in response to the voltage induced across the terminals 311 and 312. The waveform of the current I₂ will correspond to the waveform of the current I₁ and the PEMF 160 as shown in Figure 2.

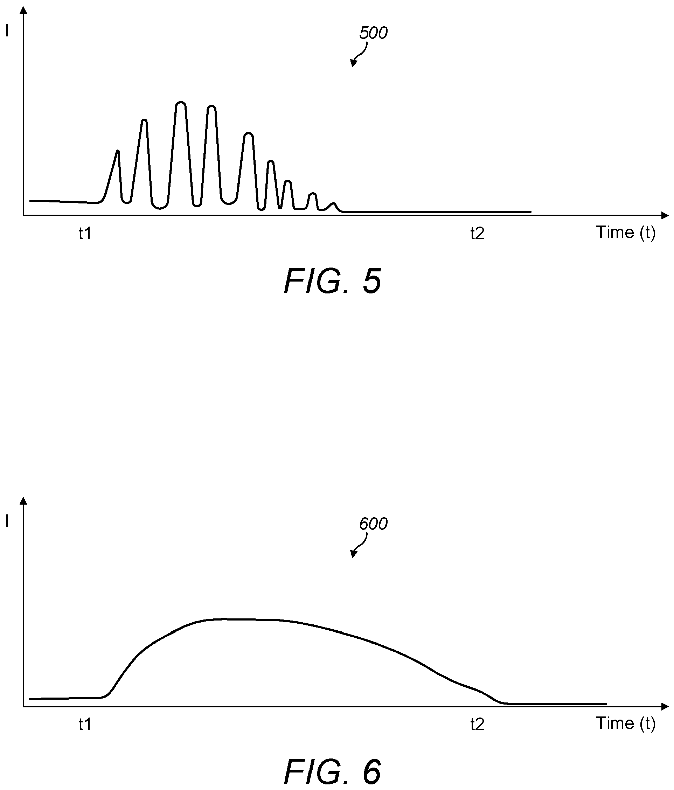

The conditioning circuit 320 receives the current I₂ from the coil loop 310 and conditions the current I₂. The conditioning circuit 320 conditions, alters and/or shapes the waveform of the current I₂. In particular the conditioning circuit 320 conditions the waveform of the current I₂ so that it is in a condition suitable for conversion into a light signal by the optical transmitter (e.g. of appropriate shape, waveform, and/or current level). The conditioning circuit 320 then outputs a conditioned current I₃ to the optical transmitter 330. The conditioned current I₃ may comprise at least a portion of the original current I₂. Figure 6 illustrates an example of a waveform 600 of the conditioned current I₃. As shown in comparison to the waveform 200 in Figure 2, the conditioning circuit 320 conditions the current I₂ and outputs a smooth pulse over the duration of a pulse of the detected PEMF (i.e. over the duration of the decaying sequence of oscillations).

As shown in Figure 3, the conditioning circuit 320 comprises a rectifier 322, a low-pass filter 324 and a suppressor and/or limiter 326. The input to the rectifier 322 is coupled to the input to the conditioning circuit 320 (i.e. to the terminals 311 and 312). The input to the low pass filter 324 is coupled to the output of the rectifier 322. The input to the suppressor / limiter 326 is coupled to the output of the low pass filter 324. The output of the suppressor / limiter 326 is coupled to the output of the conditioning circuit 320 (i.e. to the input to the optical transmitter 330).

The rectifier 322 receives the current I₂ and rectifies the current I₂. The rectifier 322 may be a half-wave rectifier, a full wave rectifier or otherwise. Figure 5 illustrates an example waveform 500 of the current at the output of the rectifier 322 when the rectifier is a half-wave rectifier. As shown, the rectifier 322 may half-wave rectify the current I₂, to only pass or permit the positive half of the current I₂. The low pass filter 324 receives the output of the rectifier 322 and performs low-pass filtering on the output of the rectifier 322. The low-pass filter is tuned to have a cut off frequency such that it applies smoothing to the output of the rectifier 322 (i.e. to the waveform 500). Consequently, the output current of the low pass filter 324 will substantially correspond to the waveform 600 in Figure 6. In particular, the output of the low pass filter 324 comprises a smooth pulse that lasts over the duration of a pulse of the pulsed EMF (e.g. between times t1 and t2). The suppressor / limiter 326 receives the output of the low-pass filter. The suppressor / limiter 326 performs signal suppression and/or limiter functions to suppress and/or limit the peak voltage and/or current at the output of the conditioning circuit 320. The suppressor / limiter 326 then outputs the current I₃ having the waveform 600 as shown in Figure 6, which is provided to the optical transmitter 330.

The optical transmitter 330 receives the output of the conditioning circuit 320. The optical transmitter 330 converts the output of the conditioning circuit 320 into a light signal and provides the light signal to the fibre optic cable 150. The light signal generated by the optical transmitter 330 has an intensity corresponding to the waveform 600 of the current I₃.

Advantageously, the PEMF sensor 140 outputs a pulsed signal (e.g. with the waveform 600) for each pulse of a detected PEMF. This enables the control circuitry 135 to clearly distinguish when a pulse of a PEMF has been detected by the PEMF sensor 140.

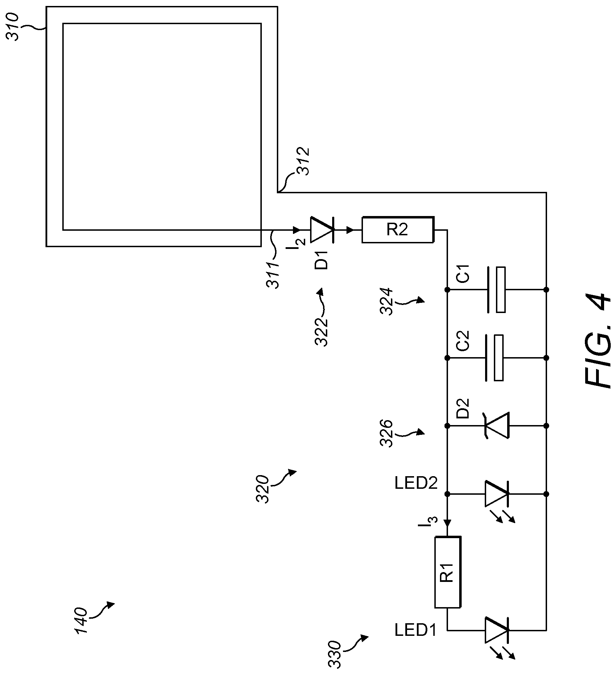

In the illustrated example of Figure 3, the coil loop 310 comprises one turn. However, in some examples, the coil loop 310 may comprise two, or more turns, for example as shown in Figures 4, 7 and 8 discussed below. The number of turns may be a design choice based on the amount of power required by the PEMF sensor 140.

Figure 4 shows a schematic of an example implementation of the PEMF sensor 140. In the example of Figure 4, the rectifier 322 comprises a diode D1. The low pass filter 324 comprises a resistor R2, a first capacitor C1 and a second capacitor C2. The suppressor/limiter 326 comprises a diode D2, a LED LED2 and a resistor R1. The optical transmitter 330 comprises a further LED LED1.

The diode D1 comprises an anode and a cathode. The anode of the diode D1 is coupled to the first terminal 311 of the coil loop 310. The cathode of the diode D1 is coupled to a first side of the resistor R2. As such, in the example of Figure 4, the diode D1 forms a half-wave rectifier. Consequently, the current I₂ will only flow in a positive direction, e.g. clockwise around the PEMF sensor 140 from the first terminal 311 to the second terminal 312. In particular, the diode D1 may output a current signal having a waveform corresponding to the half-wave rectified waveform 500 shown in Figure 5, as discussed above.

A second side of the resistor R2 is coupled to a first side of the capacitor C1 and also to a first side of the capacitor C2. The second sides of the capacitors C1 and C2 are coupled to the second terminal 312 of the coil loop 310. As such, the capacitors C1 and C2 are coupled in parallel. The capacitors C1 and C2, and the resistor R2 together form a passive low-pass filter. The cut-off frequency of the low-pass filter 324 can be tuned by choosing appropriate capacitance values of the capacitors C1 and C2, and an appropriate resistance value of the resistor R2. The cut-off frequency is preferably chosen so that the low-pass filter has a smoothing effect on the signal outputted by the diode D1, for example resulting in a smoothed pulse similar to the waveform 600 illustrated in Figure 6.

In some examples, the capacitors C1 and C2 can be replaced by a single capacitor having an equal capacitance to the parallel combination of C1 and C2. However, in some implementations, the capacitance required in the low-pass filter 324 is relatively high. To achieve the desired capacitance, the single capacitor would require a large physical volume. In particular, the required single capacitor may have a large height. Therefore, it can be advantageous to use a parallel combination of two smaller capacitors C1 and C2 to achieve the desired capacitance. Consequently, the desired capacitance can be achieved in the low pass filter 324 whilst reducing the physical height and/or volume of the circuit. This may allow for the PEMF sensor 140 to be more easily fitted into the housing 110 of the PEMF device 100. Furthermore, using a single capacitor in the low-pass filter may introduce a single point of failure for the circuit. This is especially problematic if using tantalum capacitors, which are more likely to fail. Therefore, using two capacitors C1 and C2 in parallel further provides for a more reliable circuit. For example, if one of the two capacitors C1 or C2 fail, then the circuit may still partially function to a certain degree until the circuit can be repaired.

Although Figure 4 only shows the use of two capacitors C1 and C2, it will be appreciated that any number of two or more capacitors may be coupled in parallel to achieve the desired capacitance and reduction in circuit height. Preferably, the number of capacitors and the capacitance of the capacitors are selected so that the capacitors have a height of approximately 1.8mm or less, whilst achieving the desired overall capacitance of the low pass filter.

In example implementations, the coil loop 310 may generate a high power in response to the PEMF pulse. This will mean that the capacitors C1 and C2 will charge at a very fast rate, which has the potential to damage the capacitors. Therefore, the resistor R2 further acts to limit the charge rate of the capacitors C1 and C2 to a safe and reliable level. It will be appreciated that the precise component values of the resistor R2 and the capacitors C1 and C2 are a design choice that is dependent on specific implementations of the present invention. Therefore, the skilled person may determine appropriate component values to meet the above requirements of the cut-off frequency, height and safety/reliability of the circuit.

The diode D2 comprises a cathode and an anode. The cathode of the diode D2 is coupled to the first side of the capacitors C1 and C2. The anode of the diode D2 is coupled to the second terminal 312 of the coil loop 310. The diode D2 is preferably a TVS (transient voltage suppression) diode, though any other suitable diode type may be used. The LED LED2 also comprises an anode and a cathode. The anode of LED2 is coupled to the cathode of the diode D2. The cathode of LED2 is coupled to the second terminal 312 of the coil loop 310. The LED2 is preferably a visible light LED, such as a white light LED. As such, the LED2 and the diode D2 are coupled in parallel with the capacitors C1 and C2 of the low pass filter 324. A first side of the resistor R1 is coupled to the anode of LED2 and the cathode of the diode D2. A second side of the resistor R2 is coupled to an anode of the LED1. As such, the resistor R1 is coupled in series between the LED2 / diode D2 and the LED1.

The LED1 further has a cathode. The cathode of the LED1 is coupled to the second terminal 312 of the coil loop 310. Although not shown in Figure 4, the LED1 is arranged to output its light signal to the fibre optic cable 150. Preferably, the LED1 is an infrared (IR) LED. However, the LED1 may be any other suitable type of LED for outputting a signal over a fibre optic cable.

Although the resistor R2 limits the charge rate of the capacitors C1 and C2, the voltage across the capacitors C1 and C2 may still be very high due to the high power outputted by the coil loop 310. Therefore, the diode D2 further acts to limit the voltage across the capacitors C1 and C2. In particular, the diode D2 may limit or clip the maximum voltage across the capacitors C1 and C2 during the PEMF pulse. Furthermore, the LED2 also contributes to a voltage limiting and/or clipping effect on the voltage across the capacitors C1 and C2. Consequently, the power delivered to the optical transmitter is limited or reduced. In alternative embodiments, the LED2 can be replaced with an alternative component, such as a regular diode, and achieve the same contribution to the voltage limiting and/or clipping effect.

Advantageously, the LED2 can also act as a visible indicator to the user that the PEMF sensor 140 is detecting PEMF pulses. For example, the LED2 will output a light pulse for each PEMF pulse that is detected by the coil loop 310. This allows the user to determine why the PEMF device 100 may have turned off, and may prompt the user to take the necessary actions to enable safe operation of the PEMF device 100 (e.g. by moving the coil looped inductor 120 to a new location and then restarting the PEMF device 100). Preferably, the PEMF sensor 140 is positioned or embedded in the housing 110 such that the LED2 is visible to a user external to the housing. For example, the PEMF sensor 140 can be embedded in the top surface of the housing 110 such that the LED2 is visible from the exterior of the housing.

As discussed above, the current I₃ outputted by the suppressor/limiter 326 and received by the LED1 may correspond to the waveform 600. The LED1 will therefore output a pulse of light corresponding to the waveform 600. In particular, the LED1 will output a pulse of light for each pulse of the PEMF that is detected by the coil loop 310.

The resistor R1 acts to limit the current being delivered to the LED1. Consequently, the resistor R1 will limit the sensitivity of the LED1. For example, a higher resistance R1 may result in a lower current delivered to the LED1 and therefore a lower intensity light pulse outputted by the LED1.

Preferably, the resistors R1 and R2 have resistances in the range of 1 ohm to 10 ohms. More preferably, the resistors R1 and R2 have resistances in the range of 3.3 ohms to 10 ohms. Most preferably, the resistors R1 and R2 have resistance values of approximately 4.7 ohms.

Preferably, the capacitors C1 and C2 have a combined capacitance in the range of 10 microfarads to 100 microfarads. More preferably, the capacitors C1 and C2 have a combined capacitance of approximately 20 microfarads. For example, the capacitor C1 can have a capacitance of 10 microfarads, and the capacitor C2 can have a capacitance of 10 microfarads.

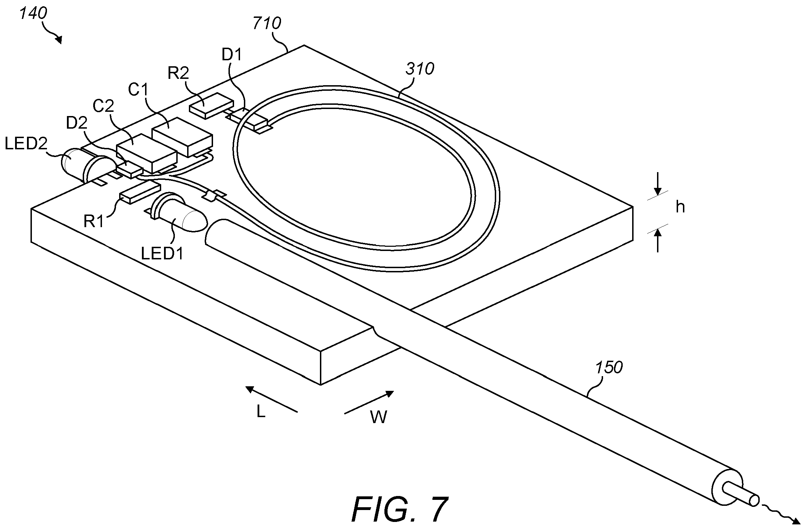

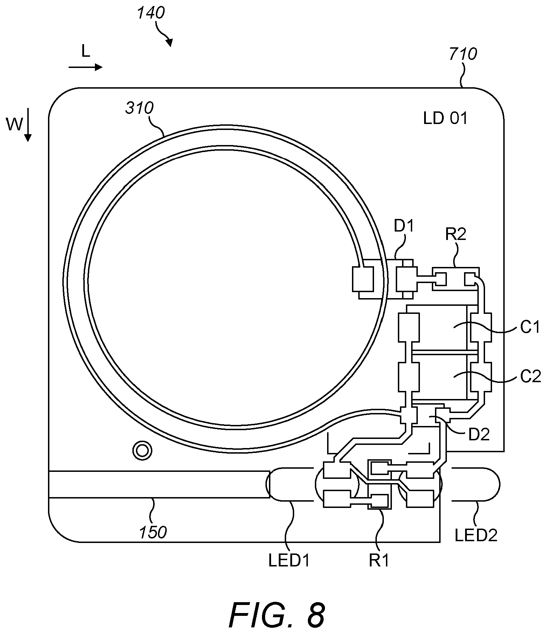

Figure 7 shows a perspective view of an implementation of the PEMF sensor circuit of Figure 4. Furthermore, Figure 8 shows a plan view of the implementation of the PEMF sensor circuit of Figure 4.

As shown in Figures 7 and 8, the components of the PEMF sensor circuit 140 in Figure 4 are implemented on a printed circuit board (PCB) 710. Figures 7 and 8 also show the arrangement of the fibre optic cable 150. As shown, the LED1 is positioned adjacent to an input end of the fibre optic cable 150 such that the light outputted by the LED1 is transmitted through the fibre optic cable 150.

Furthermore, the fibre optic cable 150 is positioned within a groove or trench of the PCB 710. In other words, the fibre optic cable 150 is recessed into the PCB 710. Advantageously, this can allow for a reduction in the height of the PEMF sensor 140. Alternatively or additionally, although not shown in Figure 7, the upper surface of the PCB 710 can be covered by a protective cover. The protective cover can have a length and width similar to that of the PCB in order to fit over the PCB 710. The protective cover can include a trench or groove for receiving the fibre optic cable 150. The protective cover can also include a recess for receiving the circuit components R1, R2, LED1, D1, D2 etc. Advantageously the trench and/or recess allows the protective cover to be a snug fit with the top surface of the PCB, providing protection to the components whilst minimising the height of the PEMF sensor 140.

The PCB 710 has a length L, width W and height h. In a preferred implementation, the PCT 710 has a length L=40mm, width W=40mm and height h=2.5mm. Advantageously, this relatively small size allows the PEMF sensor 140 to be integrated into the PEMF device 100, such as into a wall (e.g. top cover) of the housing of the PEMF device 100. However, it will be appreciated that any other dimensions of the PCB are within the scope of the present disclosure.

In the examples of Figures 7 and 8, the circuit components of the PEMF sensor 140 are provided on only one major surface of the PCB 710. As an exception, the LED2 can be positioned on an edge of the PCB 710. Advantageously, this arrangement of the circuit components ensures that the total height of the PEMF sensor 140 is minimised, allowing for easier integration of the PEMF sensor into the housing 110 of the PEMF device. Furthermore, the positioning of the LED2 may allow for the PEMF sensor 140 to be integrated into the housing 110 of the PEMF device 100 in such a way that the light outputted by the LED2 is visible from the exterior of the housing 110.

In a preferred embodiment, the fibre optic cable 150 is a polymer optical fibre. Alternatively, the fibre optic cable 150 can be a glass optical fibre. Furthermore, in a preferred implementation, the fibre optic cable 150 has a diameter of approximately 2.3mm. However, it will be appreciated that a fibre optic cable 150 of any other diameter can be used.

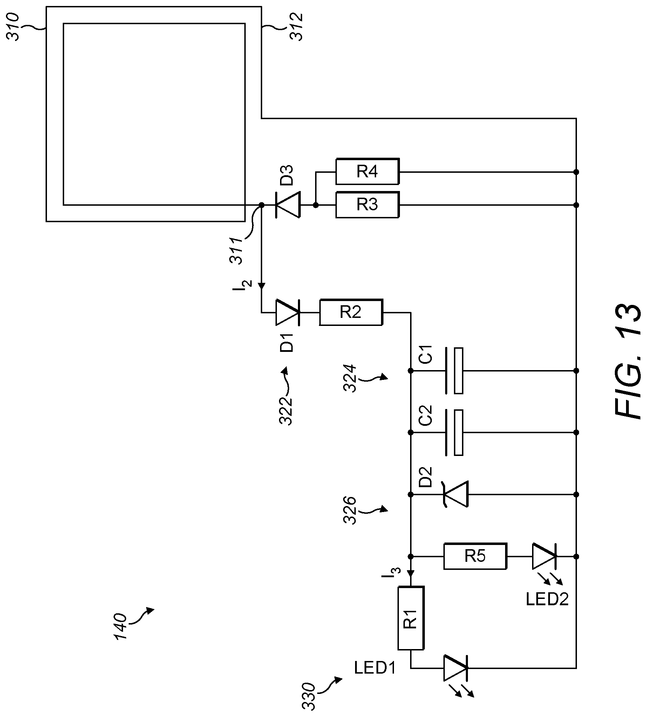

Figure 13 shows a schematic of another example implementation of the PEMF sensor 140. The example of Figure 13 illustrates a modification of the PEMF sensor 140 described in respect of Figures 4, 7 and 8. In particular, the PEMF sensor 140 shown in Figure 13 corresponds to the PEMF sensor 140 shown in Figure 4, with the following modifications or differences.

As shown in Figure 13, the PEMF sensor 140 can further comprise an additional diode D3, and additional resistors R3 and R4. A cathode of the diode D3 is coupled to the terminal 311 of the coil loop 310. I.e. the cathode of the diode D3 is coupled to the same terminal 311 to which the anode of the diode D1 is coupled. As such, the cathode of the diode D3 is coupled to the anode of the diode D1. An anode of the diode D3 is coupled to respective first sides of the resistors R3 and R4. Respective second sides of the resistors R3 and R4 are both coupled to the terminal 312 of the coil loop 310. As such, the resistors R3 and R4 are arranged in parallel between the anode of D3 and the terminal 312 of the coil 310. More generally, the diode D3 is coupled between the terminals 311 and 312 of the coil 310, whereby its cathode is coupled to the terminal 311 and the anode is coupled to the terminal 312, via the resistors R3 and R4. It should be appreciated that two parallel resistors R3 and R4 are shown as an example, and rather the resistors R3 and R4 may be replaced by an equivalent resistance using any number of resistors (e.g. one resistor, or more) in any suitable configuration. The diode D3 and the resistors R3 and R4 can be considered as part of the conditioning circuit 320, or as a separate quenching circuit that is configured to provide a current path for non-rectified portions or parts of the current induced in the coil loop (e.g. for the negative parts of the induced current).

As discussed above, the diode D1 acts as a half-wave rectifier. As such, the diode D1 passes the current I₂ (e.g. having the waveform shown in Figure 5) which corresponds to only the positive parts of the waveform of the received by the coil loop 310 (e.g. the waveform shown in Figure 2). Consequently, during the negative parts of the waveform at the coil loop 310, the diode D1 must be able to withstand an extremely large voltage (e.g. 10-100 volts or more). This has the disadvantage that the diode D1 must have very large tolerance levels, thus being large in size and more expensive.

By providing the diode D3 as shown in Figure 13, the negative parts of the waveform can be quenched via the resistors R3 and R4. Consequently, the diode D1 does not have to be able to withstand large voltages during the negative parts of the waveform. Therefore, the diode D1 can have a lower tolerance, and therefore be cheaper and smaller. This contributes to providing a smaller footprint PEMF sensor 140 that can be better integrated into the PEMF device 100. A further advantage is that the power and voltages within the PEMF sensor 140 is better managed, without requiring a full wave rectifier, the implementation of which is more complex and requires more components and circuit space.

Optionally, the PEMF sensor 140 shown in Figure 13 can include the additional resistor R5. The resistor R5 is provided in series with the LED2. In particular, the resistor R5 has a first side coupled to the anode of the LED2 and a second side coupled to the cathode of the diode D2 / the first side of the resistor R1. Advantageously, the resistor R5 acts to control and/or control the sensitivity of the LED2 by limiting the current through the LED2.

Preferably, the resistors R3, R4 and R5 have resistances in the range of 1 ohm to 10 ohms. More preferably, the resistors R3, R4 and R5 have resistances in the range of 3.3 ohms to 10 ohms. Most preferably, the resistors R3, R4 and R5 have resistance values of approximately 4.7 ohms.

It will be appreciated that the same layout principles described in respect of Figures 7 and 8 may apply for the PEMF sensor 140 shown in Figure 13, to achieve the same advantages. In particular, the components D3, R3, R4 and R5 can be provided on the same major surface of the PCB as the other components R1, R2, C1, C2, D1, D2, etc.

### Current Generating Circuit

Example implementations of the current generating circuit 130 and the coil looped inductor 120 are described as follows. In the following description, the combination of the current generating circuit 130 and the coil looped inductor 120 are referred to as PEMF generating circuitry. Although not shown in the following figures, the control circuitry 135 used to control the operations of the current generating circuit 130 may also be considered as part of the PEMF generating circuitry.

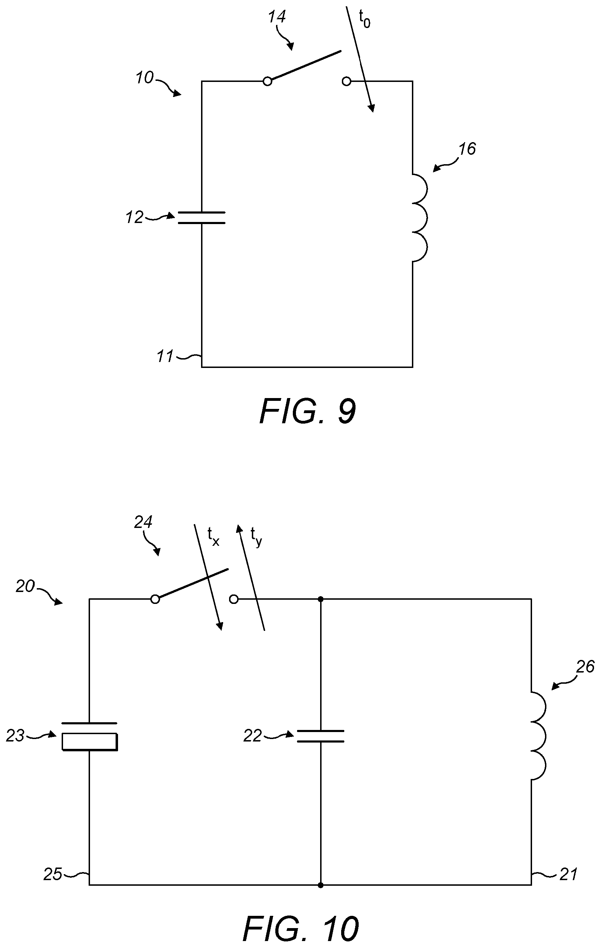

Figure 9 is a simplified circuit diagram of a first example of a PEMF generating circuit 10. The PEMF generating circuit 10 has a resonant circuit 11 with a capacitor 12 connected to a semiconductor switch 14 and a coil looped inductor 16.

When the semiconductor switch 14 is open, the capacitor 12 is charged from a high voltage circuit (not shown). Closing the semiconductor switch 14 discharges the capacitor 12 into the coil looped inductor 16, initiating oscillation of the resonant circuit 11. With the semiconductor switch 14 closed, the resonant circuit 11 oscillates until losses in the resonant circuit 11 dissipate all of the energy stored in the resonant circuit 11. Thus, when the resonant circuit 11 oscillates, a current comprising a sequence of damped or decaying sinusoidal oscillations will flow through the inductor 16. In response to the current, the inductor 16 will generate and emit a pulsed EMF correspondingly comprising a sequence of damped or decaying sinusoidal oscillations. The above process may be repeated by opening the switch 14 and then closing the switch again to generate further pulses of the pulsed EMF.

The coil looped inductor 16 can be placed adjacent to, or around, a part of the body (such as a limb or joint) where the physiological effect of the pulsed electromagnetic field is desired.

As such, the PEMF generating circuit 10 shown in Figure 9 may be used in the pulsed EMF device 100 described above, whereby the inductor 16 in Figure 9 corresponds to the coil looped inductor 120 described above and the remainder of the PEMF circuit 10 corresponds to the current generating circuit 130.

Figure 10 illustrates an example of an improved PEMF generating circuit 20.

The PEMF generating circuit 20 has a parallel resonant circuit 21 with a capacitor 22 arranged in parallel with a coil looped inductor 26. A current ramping circuit 25 is external to the parallel resonant circuit 21 and connected in parallel to the parallel resonant circuit 21. The current ramping circuit 25 includes a high current capability capacitor 23 which provides a voltage of around 50 V - 350 V (typically 150 V) and a current of around 100 A - 2000 A. A semiconductor switch 24 selectively connects the high current capability capacitor 23 to the parallel resonant circuit 21 to ramp-up the current in the coil looped inductor 26.

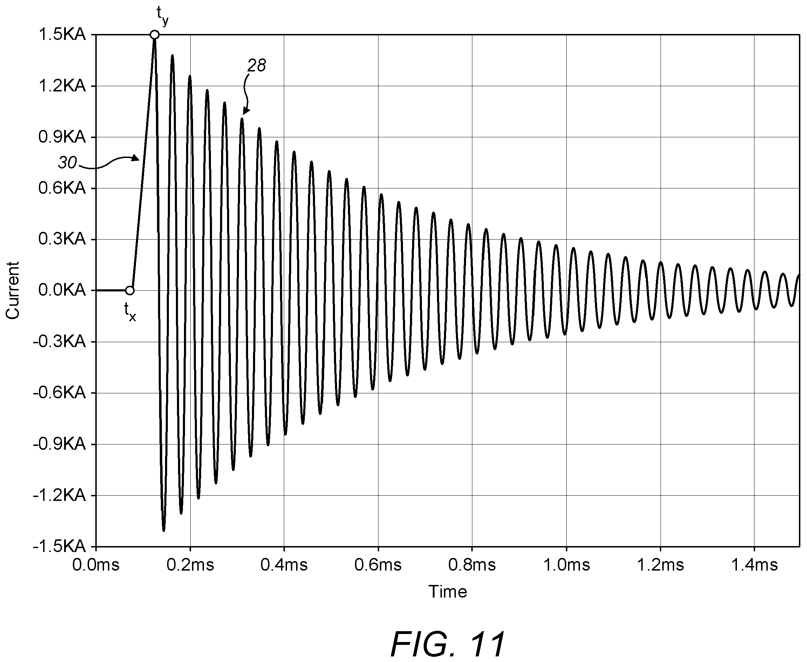

The oscilloscope trace in Figure 11 shows the current in the parallel resonant circuit 21 as a function of time. The semiconductor switch 24 is closed at *tₓ* for a current ramping period (indicated by reference numeral 30 in Figure 11) of about 50 µs to ramp-up the current in the coil looped inductor 26. At the end of the current ramping period at *t_{y}*, the semiconductor switch 24 is opened, disconnecting the current ramping circuit 25 from the parallel resonant circuit 21 and preventing further increase in the current in the coil looped inductor 26. At the end of the current ramping period, the current in the coil looped inductor 26 has reached a desired current of 1500 A, which is sufficient to produce a pulsed EMF that provides a physiological effect.

At the end of the current ramping period at *t_{y}*, and with the semiconductor switch 24 open, the current in the coil looped inductor 26 initiates oscillation of the parallel resonant circuit 21. As illustrated by the oscilloscope trace in Figure 11, the parallel resonant circuit 21 generates a pulsed EMF comprising a sequence of damped sinusoidal oscillations 28 in the coil looped inductor 26. The coil looped inductor 26 is placed adjacent to, or around, a part of the body (such as a limb or joint) where the physiological effect of the pulsed electromagnetic field is desired.

The parallel resonant circuit 21 oscillates until losses in the parallel resonant circuit 21 dissipate all of the energy stored in the parallel resonant circuit 21. To generate more pulses of the pulsed EMF, the above process may be repeated by closing the switch 24 for another current ramping period, and then opening the switch 24.

Advantageously, the semiconductor switch 24 does not need to be a component of the parallel resonant circuit 21 in order to control current within the coil looped inductor 26. Instead, current ramping of the parallel resonant circuit 21 is controlled by current ramping circuit 25 which is external to and connected in parallel to the parallel resonant circuit 21. Not having a semiconductor switch 14 as a component of the parallel resonant circuit 21 provides a number of benefits.

Resistance losses in the parallel resonant circuit 21 are low because the semiconductor switch 24 is external to the parallel resonant circuit 21, so resistance losses from the semiconductor switch 24 are not incurred during oscillation of the parallel resonant circuit 21. As a result, the decay time of the damped oscillations is much longer which increases the time period over which the pulsed electromagnetic field provides a physiological effect for a given initial current in the coil looped inductor 26. For example, a physiological effect may be present when the current in the parallel resonant circuit 21 is greater than around 200A, and the PEMF generating circuit 20 enjoys a period of around 1100 µs in which the current in the parallel resonant circuit 21 is providing a physiological effect, as compared with only 60 µs with the PEMF generating circuit 10. As a result, the PEMF generating circuit 20 provides a more sustained physiological effect. Moreover, the coil looped inductor 26 need only be ramped to a lower initial current (only 200 A - 1500 A in the pulsed electromagnetic field therapy device 20 as compared with 2000 A - 3000 A in the pulsed electromagnetic field therapy device 10), leading to lower voltages in the PEMF circuit 20 which do not require capacitor 22 or semiconductor switch 24 to be expensive high voltage components, reducing manufacturing costs. Additionally, operating at lower voltages allows capacitor 22 to have a larger capacitance value than a higher voltage capacitor of equivalent physical size, and the selection of a larger capacitance value for capacitor 22 leads to parallel resonant circuit 21 having a lower resonant frequency which allows the PEMF generating circuit 20 to meet regulatory requirements regarding electromagnetic interference.

In the PEMF generating circuit 10, the charge from the capacitor 12 is dumped into the resonant circuit 11 nearly instantaneously when the semiconductor switch 14 in the resonant circuit 11 is closed. This rapid charge discharged into the resonant circuit 11 leads to current reflections which result in significant interference 19. By not having semiconductor switch 24 as a component of the parallel resonant circuit 21, the current in the parallel resonant circuit 21 is increased more gradually over the course of the current ramping period 30. This, combined with the fact that the semiconductor switch 24 is external to and disconnected from the parallel resonant circuit 24 after the current ramping period 30 so that the impedance mismatched semiconductor switch 24 does not lead to reflections, results in a current profile in the parallel resonant circuit 21 which is sinusoidal with low distortion, and which does not show the large amount of interference 19 that may be seen in the PEMF generating circuit 10.

The PEMF generating circuit 20 shown in Figure 10 may be used in the pulsed EMF device 100 described above, whereby the inductor 26 in Figure 10 corresponds to the coil looped inductor 112 described above and the remainder of the PEMF generating circuit 20 corresponds to the current generating circuit 130.

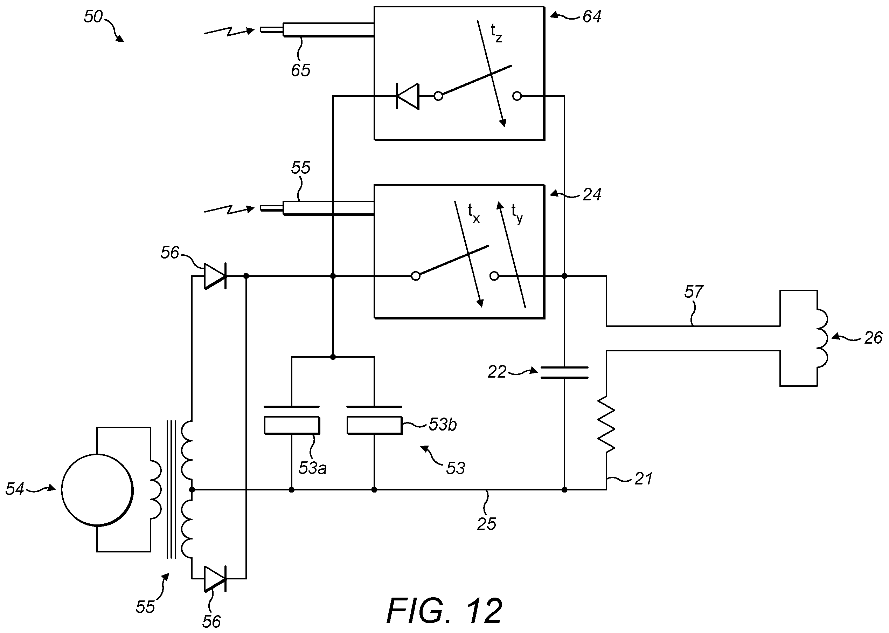

Figure 12 illustrates an alternative example of an improved PEMF generating circuit 50. The PEMF generating circuit 50 is generally the same as the pulsed electromagnetic field therapy device 20, with some improvements to electrical safety, charging and control.

The pulsed electromagnetic field may show no significant physiological effect once the current in the parallel resonant circuit 21 has dropped below a certain current (for example, once the current in the parallel resonant circuit 21 has dropped below 200 A). Therefore, a current threshold may be selected based on a current below which little or no significant physiological effect is observed, or below which insufficient physiological effect is observed to meet the needs of a particular physiological or therapeutic application.

Once the current in the parallel resonant circuit 21 has dropped below the current threshold (at a time *t_{z}* following time t_{y}), a further switch 64 is closed which connects the parallel resonant circuit 21 to the capacitor bank 53. This substantially reduces oscillation of the parallel resonant circuit 21 and allows at least part of the energy remaining in the parallel resonant circuit 21 to be recycled to at least partially recharge the capacitor bank 53. This saves considerable energy that might otherwise be wasted generating a pulsed electromagnetic field which provides no physiological effect.

Instead of a single high current capability capacitor 23, the pulsed electromagnetic field therapy device 50 has a capacitor bank 53 which is made up of capacitors 53a and 53b connected in parallel which together offer a high current capability source. The use of capacitor bank 53 may provide redundancy in case a capacitor 53a or 53b fails, and may be cheaper than using a single high current capability capacitor 23. The capacitor bank 53 could provide a high current capability source using more than two capacitors. In fact, it may be beneficial for the capacitor bank 53 to combine a large number of cheap, lower value capacitors which are smaller and therefore easier to pack into spare space in a housing.

The capacitor bank 53 is charged from power source 54. In some examples, the power source 54 is fed from a mains electricity supply. However, the power supply 54 may be any electrical power source, such as a mains power supply or a battery. To improve electrical safety, and reduce the risk of a patient or operator receiving an electrical shock from the high voltages and currents present in the current ramping circuit 25 and the parallel resonant circuit 21, the current ramping circuit 25 and the parallel resonant circuit 21 are galvanically isolated from the power source 54 by transformer 55. The transformer 55 is provided with diodes 56 for rectification purposes. Therefore, the inductor 26 and other components of the parallel resonant circuit 21 are floating, and therefore safe to touch even if insulation surrounding the inductor 26, cable 57 or other components is damaged.

To complete the isolation, the semiconductor switch 24 receives switching signals over a fibre optic cable 55 and the optional further switch 64 receives switching signals over a fibre optic cable 65. This helps to reduce induced interference which might occur on an electrical link.

The PEMF generating circuit 50 shown in Figure 12 may be used as the pulsed EMF device 100 described above, whereby the inductor 26 in Figure 12 corresponds to the coil looped inductor 120 described above and the remainder of the PEMF circuit 50 corresponds to the current generating circuit 130.

### Other Variations

In the above description, it is described that the current I₁ in the PEMF device 110 comprises a sequence of decaying or damped sinusoidal oscillations. However, in alternative examples, the current I₁ may not necessarily comprise sinusoidal oscillations. In particular, the current I₁ may comprise a sequence of decaying or damped oscillations of a non-sinusoidal shape. For example, the current I₁ may comprise a sequence of decaying or damped oscillations having a square, triangular, saw-tooth, or any other shaped oscillating waveform. Consequently, it will be appreciated that pulsed EMF 160 may have a corresponding non-sinusoidal shape, as will the voltages induced across the coil loops 310.

In some examples, the diode D1 in the PEMF sensor 140 may be replaced by a full-wave rectifier. For example, the full-wave rectifier may be a bridge-rectifier. In one example arrangement, the bridge-rectifier may have a first input terminal coupled to the terminal 311 of the coil loop 310, a second input terminal coupled to the terminal 312 of the coil loop 310, a first output terminal coupled to the first side of the resistor D1, and a second output terminal coupled to the second sides of the capacitors C1 and C2. The bridge-rectifier may comprise four diodes arranged between the input and the output terminals in a bridge-rectifier configuration. For example: an anode of a first diode is coupled to the first input terminal; a cathode of the first diode is coupled to the first output terminal; an anode of a second diode is coupled to the second output terminal; a cathode of the second diode is coupled to the first input terminal; a cathode of a third diode is coupled to the first input terminal; an anode of the third diode is coupled to the second output terminal; a cathode of a fourth diode is coupled to the second input terminal; an anode of the fourth diode is coupled to the second output terminal.

## Claims

1. A pulsed electromagnetic field device (100) comprising:
a pulsed electromagnetic field generator configured to generate electromagnetic field pulses (160); and
a sensor (140) having an output in communication with the pulsed electromagnetic field generator, wherein the sensor (140 )is configured to detect an electromagnetic field pulse (160) and provide, over the output, an output signal indicative of each detected electromagnetic field pulse (160),
**characterised in that** the pulsed electromagnetic field generator is further configured to stop generating the electromagnetic field pulses (160) in response to the sensor detecting one or more electromagnetic field pulses (160).

2. The device of claim 1, wherein each electromagnetic field pulse (160) comprises a decaying sequence of electromagnetic oscillations.

3. The device of either of claims 1 or 2, wherein the output of the sensor is in communication with the pulsed electromagnetic field generator over a fibre optic cable, and the output signal of the sensor is a light signal.

4. The device of claim 3, wherein the sensor comprises:
a coil loop configured to induce a current in response to the electromagnetic field pulse,
a conditioning circuit coupled to the coil loop, wherein the conditioning circuit is configured to condition a waveform of the induced current and output a conditioned current, and
an optical transmitter configured to convert the conditioned current into the light signal and supply the light signal to the fibre optic cable.

5. The device of claim 4, wherein the conditioning circuit comprises:
a rectifier configured to at least partially rectify the induced current and output a rectified current;
a low pass filter configured to filter the rectified current; and
a suppression circuit that is configured to limit a current and/or a voltage in the conditioning circuit;
optionally, wherein the rectifier comprises at least one diode;
the low pass filter comprises a first resistor and at least one capacitor arranged in a low pass filter configuration, wherein the first resistor is arranged in series with the diode; and
the suppression circuit comprises a transient voltage suppression diode arranged in parallel with the capacitor, a first LED arranged in parallel with the transient voltage suppression diode, and a second resistor arranged in series with an output of the suppression circuit.

6. The device of claim 5, wherein the sensor further comprises a quenching circuit configured to provide a current path for non-rectified portions of the induced current, optionally wherein the quenching circuit comprises a second diode and a resistance arranged in series between terminals of the coil loop.

7. The device of claim 5 or claim 6, wherein the first LED is a white light LED and/or wherein the optical transmitter comprises an infrared LED.

8. The device of any of claims 3 to 7, wherein the fibre optic cable is a polymer optical fibre.

9. The device of any preceding claim, wherein the pulsed electromagnetic field generator comprises:
a current generating circuit configured to generate a pulsed current;
a coil looped inductor configured to output the electromagnetic field pulses in response to the pulsed current; and
control circuitry configured to control the current generating circuit to generate the pulsed current,
wherein the control circuitry is configured to receive the output signal from the sensor and stop the current generating circuit from generating the pulsed current in response to the sensor detecting one or more electromagnetic field pulses;
optionally, wherein the pulsed electromagnetic field device further comprises a housing, the housing containing at least the current generating circuit and the control circuitry of the pulsed electromagnetic field generator, wherein the sensor is embedded in a surface of the housing.

10. A method comprising:
generating, using a pulsed electromagnetic field generator, a plurality of electromagnetic field pulses (160);
detecting, using a sensor (140), one or more electromagnetic field pulses (160);
communicating, from the sensor (140) to the pulsed electromagnetic field generator, an output signal indicative of each detected electromagnetic field pulse (160); and
the method is **characterised by** further comprising, in response to the sensor (140) detecting the one or more electromagnetic field pulses (160), stopping the pulsed electromagnetic field generator from generating further electromagnetic field pulses (160).

11. The method of claim 10, wherein the output signal is a light signal and the light signal is communicated from the sensor (140) to the pulsed electromagnetic field generator via a fibre optic cable.

12. The method of claim 11, wherein detecting the one or more electromagnetic field pulses comprises:
inducing a current in a coil loop in response to the electromagnetic field pulse;
conditioning a waveform of the induced current and outputting a conditioned current using a conditioning circuit; and
converting the conditioned current into the light signal using an optical transmitter, and supplying the light signal to the fibre optic cable.

13. The method of claim 12, wherein conditioning the waveform of the induced current comprises:
at least partially rectifying the induced current using a rectifier to produce a rectified current;
low pass filtering the rectified current; and
limiting a current and/or a voltage at the output of the conditioning circuit.

14. The method of claim 13, further comprising quenching non-rectified parts of the induced current by providing a current path for said non-rectified parts.

15. The method of any of claims 11 to 14, wherein generating the electromagnetic field pulses using the pulsed electromagnetic field generator comprises:
controlling a current generating circuit using control circuitry to generate a pulsed current from the current generating circuit; and
outputting the electromagnetic field pulses from a coil looped inductor in response to the pulsed current,
wherein the control circuitry receives the output signal from the sensor and stops the current generating circuit from generating the pulsed current in response to the sensor detecting one or more electromagnetic field pulses.

## Patentansprüche

1. Gepulste elektromagnetische Feldvorrichtung (100), umfassend:
einen gepulsten elektromagnetischen Feldgenerator, um elektromagnetische Feldimpulse (160) zu erzeugen, und
einen Sensor (140), der einen Ausgang aufweist, der mit dem gepulsten elektromagnetischen Feldgenerator in Verbindung steht, wobei der Sensor (140) dazu beschaffen ist, einen elektromagnetischen Feldimpuls (160) zu erfassen und über seinen Ausgang ein Ausgangssignal auszugeben, das für jeden erfassten elektromagnetischen Feldimpuls (160) hinweisend ist,
**dadurch gekennzeichnet, dass** der gepulste elektromagnetische Feldgenerator ferner dazu beschaffen ist, die Erzeugung der elektromagnetischen Feldimpulse (160) in Reaktion darauf, dass der Sensor einen oder mehrere elektromagnetische Feldimpulse (160) erfasst, zu stoppen.

2. Vorrichtung nach Anspruch 1, wobei jeder elektromagnetische Feldimpuls (160) eine abklingende Folge elektromagnetischer Schwingungen umfasst.

3. Vorrichtung nach einem der Ansprüche 1 oder 2, wobei der Ausgang des Sensors über einen Lichtwellenleiter mit dem gepulsten elektromagnetischen Feldgenerator in Verbindung steht und das Ausgangssignal des Sensors ein Lichtsignal ist.

4. Vorrichtung nach Anspruch 3, wobei der Sensor umfasst:
eine Spulenschleife, die dazu beschaffen ist, in Reaktion auf den elektromagnetischen Feldimpuls einen Strom zu induzieren,
eine Aufbereitungsschaltung, die mit der Spulenschleife gekoppelt ist, wobei die Aufbereitungsschaltung so konfiguriert ist, dass sie die Wellenform des induzierten Stroms aufbereitet und einen aufbereiteten Strom ausgibt, und
einen optischen Sender, der dazu beschaffen ist, den aufbereiteten Strom in ein Lichtsignal umzuwandeln und dieses Lichtsignal an den Lichtwellenleiter zu liefern.

5. Vorrichtung nach Anspruch 4, wobei die Aufbereitungsschaltung umfasst:
einen Gleichrichter, der dazu beschaffen ist, den induzierten Strom zumindest teilweise gleichzurichten und einen gleichgerichteten Strom auszugeben,
einen Tiefpassfilter, der dazu beschaffen ist, den gleichgerichteten Strom zu filtern, und
eine Unterdrückungsschaltung, die dazu beschaffen ist, einen Strom und/oder eine Spannung in der Aufbereitungsschaltung zu begrenzen,
wobei optional der Gleichrichter mindestens eine Diode umfasst,
der Tiefpassfilter einen ersten Widerstand und mindestens einen Kondensator umfasst, die in einer Tiefpassfilterkonfiguration angeordnet sind, wobei der erste Widerstand in Reihe mit der Diode geschaltet ist, und
die Unterdrückungsschaltung eine Diode zur Unterdrückung transienter Spannungen, die parallel zum Kondensator geschaltet ist, eine erste LED, die parallel zur Diode zur Unterdrückung transienter Spannungen geschaltet ist, und einen zweiten Widerstand, der in Reihe mit dem Ausgang der Unterdrückungsschaltung geschaltet ist, umfasst.

6. Vorrichtung nach Anspruch 5, wobei der Sensor ferner eine Löschschaltung umfasst, die einen Strompfad für nicht gleichgerichtete Anteile des induzierten Stroms bereitstellt, wobei die Löschschaltung optional eine zweite Diode und einen Widerstand umfasst, die in Reihe zwischen den Anschlüssen der Spulenschleife geschaltet sind.

7. Vorrichtung nach Anspruch 5 oder 6, wobei die erste LED eine weiße LED ist und/oder wobei der optische Sender eine Infrarot-LED umfasst.

8. Vorrichtung nach einem der Ansprüche 3 bis 7, wobei der Lichtwellenleiter eine Polymer-Lichtleitfaser ist.

9. Vorrichtung nach einem der vorhergehenden Ansprüche, wobei der gepulste elektromagnetische Feldgenerator umfasst:
eine Stromerzeugungsschaltung zur Erzeugung eines gepulsten Stroms,
eine Spuleninduktivität zur Ausgabe von elektromagnetischen Feldimpulsen in Abhängigkeit vom gepulsten Strom und
eine Steuerschaltung zur Steuerung der Stromerzeugungsschaltung zur Erzeugung des gepulsten Stroms,
wobei die Steuerschaltung dazu beschaffen ist, das Ausgangssignal vom Sensor zu empfangen und die Stromerzeugungsschaltung zu stoppen, den gepulsten Strom in Reaktion darauf zu stoppen, dass der Sensor einen oder mehrere elektromagnetische Feldimpulse erfasst,
wobei optional die gepulste elektromagnetische Feldvorrichtung ferner ein Gehäuse umfasst, das mindestens die Stromerzeugungsschaltung und die Steuerschaltung des Impulsgenerators enthält, wobei der Sensor in eine Oberfläche des Gehäuses eingebettet ist.

10. Verfahren umfassend:
Erzeugen, unter Verwendung eines gepulsten elektromagnetischen Feldgenerators, einer Vielzahl von elektromagnetischen Feldimpulsen (160),
Erfassen, unter Verwendung eines Sensors (140), eines oder mehrerer elektromagnetischer Feldimpulse (160),
Kommunizieren, vom Sensor (140) an den gepulsten elektromagnetischen Feldgenerator, eines Ausgangssignals, das für jeden erfassten elektromagnetischen Feldimpuls (160) hinweisend ist, und
das Verfahren **dadurch gekennzeichnet ist, dass** es ferner in Reaktion darauf, dass der Sensor (140) einen oder mehrere elektromagnetische Feldimpulse (160) erfasst, umfasst, dass der gepulste elektromagnetische Feldgenerator die Erzeugung weiterer elektromagnetischer Feldimpulse (160) stoppt.

11. Verfahren nach Anspruch 10, wobei das Ausgangssignal ein Lichtsignal ist und dieses Lichtsignal vom Sensor (140) über einen Lichtwellenleiter an den gepulsten elektromagnetischen Feldgenerator übertragen wird.

12. Verfahren nach Anspruch 11, wobei das Erfassen des einen oder der mehreren elektromagnetischer Feldimpulse umfasst:
Induzieren eines Stroms in einer Spulenschleife als Reaktion auf den elektromagnetischen Feldimpuls,
Aufbereiten einer Wellenform des induzierten Stroms und Ausgeben eines aufbereiteten Stroms unter Verwendung einer Aufbereitungsschaltung und
Umwandeln des aufbereiteten Stroms in das Lichtsignal unter Verwendung eines optischen Senders und Zuführen des Lichtsignals zum Lichtwellenleiter.

13. Verfahren nach Anspruch 12, wobei die Konditionierung der Wellenform des induzierten Stroms umfasst:
Zumindest teilweise Gleichrichten des induzierten Stroms unter Verwendung eines Gleichrichters zum Erzeugen eines gleichgerichteten Stroms,
Tiefpassfiltern des gleichgerichteten Stroms und
Begrenzen eines Stroms und/oder einer Spannung am Ausgang der Aufbereitungsschaltung.

14. Verfahren nach Anspruch 13, ferner umfassend das Löschen nicht gleichgerichteter Anteile des induzierten Stroms durch Bereitstellung eines Strompfads für diese Anteile.

15. Verfahren nach einem der Ansprüche 11 bis 14, wobei das Erzeugen der elektromagnetischen Feldimpulse unter Verwendung des gepulsten elektromagnetischen Feldgenerators umfasst:
Steuern einer Stromerzeugungsschaltung unter Verwendung einer Steuerschaltung, um einen gepulsten Strom aus der Stromerzeugungsschaltung zu erzeugen, und
Ausgeben von elektromagnetischen Feldimpulsen aus einer Spulenschleife als Reaktion auf den Impulsstrom,
wobei die Steuerschaltung das Ausgangssignal des Sensors empfängt und die Stromerzeugungsschaltung in Reaktion darauf, dass der Sensor einen oder mehrere elektromagnetische Feldimpulse erfasst, stoppt, den gepulsten Strom zu erzeugen.

## Revendications

1. Un dispositif à champ électromagnétique pulsé (100) comprenant :
un générateur de champ électromagnétique pulsé configuré pour générer des impulsions de champ électromagnétique (160) ; et
un capteur (140) ayant une sortie en communication avec le générateur de champ électromagnétique pulsé, dans lequel le capteur (140) est configuré pour détecter une impulsion de champ électromagnétique (160) et fournir, sur la sortie, un signal de sortie indicatif de chaque impulsion de champ électromagnétique détectée (160),
**caractérisé en ce que** le générateur de champ électromagnétique pulsé est en outre configuré pour arrêter de générer les impulsions de champ électromagnétique (160) en réponse à la détection par le capteur d'une ou plusieurs impulsions de champ électromagnétique (160).

2. Le dispositif selon la revendication 1, dans lequel chaque impulsion de champ électromagnétique (160) comprend une séquence décroissante d'oscillations électromagnétiques.

3. Le dispositif selon l'une des revendications 1 ou 2, dans lequel la sortie du capteur est en communication avec le générateur de champ électromagnétique pulsé sur un câble à fibre optique, et le signal de sortie du capteur est un signal lumineux.

4. Le dispositif selon la revendication 3, dans lequel le capteur comprend :
une boucle de bobine configurée pour induire un courant en réponse à l'impulsion de champ électromagnétique,
un circuit de conditionnement couplé à la boucle de bobine, dans lequel le circuit de conditionnement est configuré pour conditionner une forme d'onde du courant induit et délivrer un courant conditionné, et
un émetteur optique configuré pour convertir le courant conditionné en signal lumineux et fournir le signal lumineux au câble à fibres optiques.

5. Le dispositif selon la revendication 4, dans lequel le circuit de conditionnement comprend :
un redresseur configuré pour redresser au moins partiellement le courant induit et délivrer un courant redressé ;
un filtre passe-bas configuré pour filtrer le courant redressé ; et
un circuit de suppression qui est configuré pour limiter un courant et/ou une tension dans le circuit de conditionnement ;
éventuellement, dans lequel le redresseur comprend au moins une diode ;
le filtre passe-bas comprend une première résistance et au moins un condensateur disposés dans une configuration de filtre passe-bas, la première résistance étant disposée en série avec la diode ; et
le circuit de suppression comprend une diode de suppression de tension transitoire disposée en parallèle avec le condensateur, une première LED disposée en parallèle avec la diode de suppression de tension transitoire, et une deuxième résistance disposée en série avec une sortie du circuit de suppression.

6. Le dispositif selon la revendication 5, dans lequel le capteur comprend en outre un circuit d'extinction configuré pour fournir un trajet de courant pour des parties non redressées du courant induit, éventuellement dans lequel le circuit d'extinction comprend une deuxième diode et une résistance disposées en série entre les bornes de la boucle de bobine.

7. Le dispositif selon la revendication 5 ou la revendication 6, dans lequel la première LED est une LED à lumière blanche et/ou dans lequel l'émetteur optique comprend une LED infrarouge.

8. Le dispositif selon l'une quelconque des revendications 3 à 7, dans lequel le câble à fibre optique est une fibre optique polymère.

9. Le dispositif selon l'une quelconque des revendications précédentes, dans lequel le générateur de champ électromagnétique pulsé comprend :
un circuit de génération de courant configuré pour générer un courant pulsé ;
un inducteur à bobine en boucle configuré pour émettre les impulsions de champ électromagnétique en réponse au courant pulsé ; et
des circuits de commande configurés pour commander le circuit de génération de courant afin de générer le courant pulsé,
dans lequel le circuit de commande est configuré pour recevoir le signal de sortie du capteur et empêcher le circuit de génération de courant de générer le courant pulsé en réponse à la détection par le capteur d'une ou plusieurs impulsions de champ électromagnétique ;
éventuellement, dans lequel le dispositif à champ électromagnétique pulsé comprend en outre un boîtier, le boîtier contenant au moins le circuit de génération de courant et le circuit de commande du générateur de champ électromagnétique pulsé, dans lequel le capteur est intégré dans une surface du boîtier.

10. Procédé comprenant les étapes consistant à :
générer, à l'aide d'un générateur de champ électromagnétique pulsé, une pluralité d'impulsions de champ électromagnétique (160) ;
détecter, à l'aide d'un capteur (140), une ou plusieurs impulsions de champ électromagnétique (160) ;
communiquer, du capteur (140) au générateur de champ électromagnétique pulsé, un signal de sortie indicatif de chaque impulsion de champ électromagnétique détectée (160) ; et
le procédé est **caractérisé en ce qu'**il comprend en outre, en réponse à la détection par le capteur (140) de la ou des impulsions de champ électromagnétique (160), l'arrêt de la génération d'autres impulsions de champ électromagnétique (160) par le générateur de champ électromagnétique pulsé.

11. Le procédé selon la revendication 10, dans lequel le signal de sortie est un signal lumineux et le signal lumineux est communiqué du capteur (140) au générateur de champ électromagnétique pulsé via un câble à fibre optique.

12. Le procédé selon la revendication 11, dans lequel la détection de la ou des impulsions de champ électromagnétique comprend les étapes consistant à :
induire un courant dans une boucle de bobine en réponse à l'impulsion de champ électromagnétique ;
conditionner une forme d'onde du courant induit et émettre un courant conditionné à l'aide d'un circuit de conditionnement ; et
convertir le courant conditionné en signal lumineux à l'aide d'un émetteur optique, et fournir le signal lumineux au câble à fibre optique.

13. Procédé selon la revendication 12, dans lequel le conditionnement de la forme d'onde du courant induit comprend les étapes consistant à :
redresser au moins partiellement le courant induit à l'aide d'un redresseur pour produire un courant redressé ;
filtrer en passe-bas le courant redressé ; et
limiter un courant et/ou une tension à la sortie du circuit de conditionnement.

14. Procédé selon la revendication 13, comprenant en outre l'extinction des parties non redressées du courant induit en fournissant un trajet de courant pour lesdites parties non redressées.

15. Procédé selon l'une quelconque des revendications 11 à 14, dans lequel la génération des impulsions de champ électromagnétique à l'aide du générateur de champ électromagnétique pulsé comprend les étapes consistant à :
commander un circuit de génération de courant à l'aide d'un circuit de commande pour générer un courant pulsé à partir du circuit de génération de courant ; et
émettre les impulsions de champ électromagnétique à partir d'une inductance en boucle de bobine en réponse au courant pulsé,
dans lequel le circuit de commande reçoit le signal de sortie du capteur et empêche le circuit de génération de courant de générer le courant pulsé en réponse à la détection par le capteur d'une ou plusieurs impulsions de champ électromagnétique.
